# EUROPEAN PATENT APPLICATION

(11) **EP 1 645 294 A2**
(43) Date of publication of application: **12.04.2006**
(21) Application number: 05021707.4
(22) Date of filing: 05.10.2005
(51) Int. Cl.: A61L 9/12, A61L 9/03, A61L 9/14

(54) **Fragrance diffuser**

(30) Priority: 08.10.2004 IT MI20040453
(71) Applicant: OIKOS S.r.l., 20129 Milano (IT)
(72) Inventor: Bader, Stefano, 20121 Milano (IT); Verderio, Silvia, 20121 Milano (IT)
(74) Representative: Minoja, Fabrizio

(57) **Abstract**

Disclosed is an appliance designed for mixing perfumed essences and diffusing them in a room, characterised in that it includes a common support or frame (2) with which are associated a number of diffusers (1) each comprising a fragrance container (4) and means (6) designed to diffuse said fragrance in the room simultaneously with the others, at an adjustable intensity.

## Description

This innovation relates to a device designed to mix perfumed essences and diffuse them in a room, which said device comprises, mounted on a single frame, a plurality of cartridges each containing a different perfuming product, each of which cartridges is associated with means designed to direct onto the product, with adjustable intensity, an airstream that flows over the perfuming product and is then conveyed into the room.

More particularly, each cartridge is associated with a fan which can be activated at different speeds or different time intervals to vary the intensity of the fragrance conveyed into the room, thus enabling the fragrances to be modulated and mixed as required to create a wide range of perfumes.

The power supply of said fan can be connected to a dedicated control unit with controls and timers, or to the units that control the operation of the other electrical and electronic devices in the home (home automation control units), to regulate the emission of perfume as required.

Different types of diffuser are known which release a perfume or deodorant into a location which could be a room of a home, a vehicle interior or the like.

Many of these devices diffuse the perfume into the room by evaporation of the product.

As the fragrances are generally constituted by oils which have a fairly high boiling point and/or vapour pressure, in order to allow evaporation at a temperature just above room temperature, said oils are mixed with solvents, producing a liquid composition which is used in association with diffusers constituted by a container fitted with a resistor that heats the composition gently to promote dispersal of the perfume into the room.

These are basically devices wherein a complex formulation is caused to evaporate by means of kinetics due to a change of state resulting from a temperature increase, with the result that evaporation and the extent of vapour diffusion cannot be easily controlled or modulated.

These diffusers need to be very cheap; it is not economical to equip them with temperature control devices or sensors, so a low-powered resistor that always develops the same amount of heat is fitted.

If the container contains a small amount of product, the product will reach a higher temperature, diffusing a larger quantity of perfume than when the container is full of liquid.

The diffusion of the perfume also depends on other factors, such as environmental humidity, etc., which not are easily controllable.

Another type of diffuser diffuses the perfume into the room by spraying a liquid that contains the fragrance.

Part of the liquid remains suspended in the air and part falls to the ground, with the result that the effect and extent of perfuming varies, depending on whether people are moving about in the room, for example.

Systems are also known which involve sprinkling into the room microcapsules containing perfume that break when they are trodden on, releasing the fragrance.

Once again, the effect is obviously not controllable.

Basically, systems which involve diffusion of a perfume mixed with a solvent cannot easily be modulated, because even if the diffusion of the solvent in the air could be controlled, it is difficult to control how the solvent releases the fragrance.

Moreover, with known systems the choice of fragrances available to users is limited, and it is impossible to modulate their intensity, modify and/or mix the perfumes.

The present innovation, which falls into this sector, relates to a modulator/mixer/diffuser designed to diffuse in a room a mixture of different fragrances, in variable proportions, which can be selected and modified by the user.

For this purpose, the appliance according to the invention comprises a set of cartridges each containing a different fragrance, each cartridge being associated with a fan that diffuses the fragrance in the room and can be switched on or off and operated at a fixed or variable speed, so that a single fragrance or a mixture of two or more fragrances is diffused in the room in the preferred proportions.

This innovation will now be described in detail, by way of example but not of limitation, by reference to the annexed figures wherein:
- figure 1 is a front view of a diffuser according to the invention, wall-mounted;
- figure 2 is the perspective view of the support frame of the diffusers in a device according to the invention;
- figure 3 shows the side view of a device according to the invention, assembled.

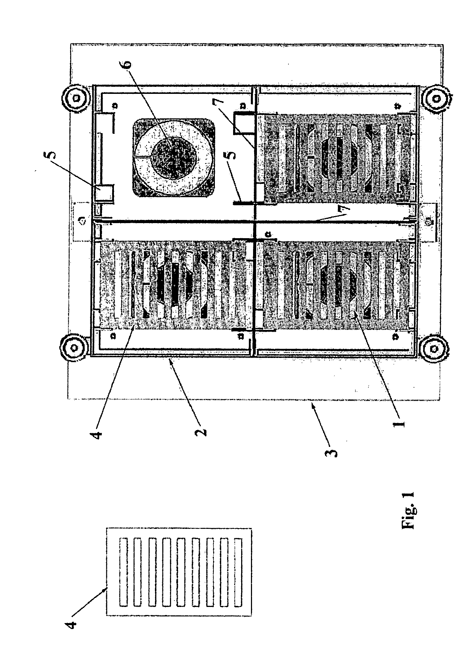

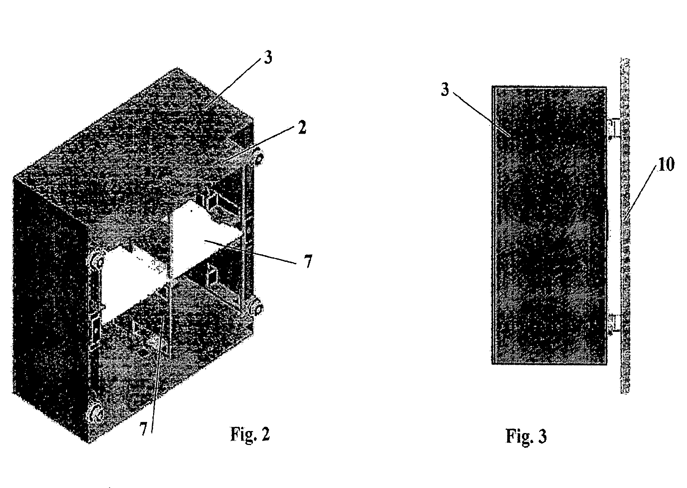

As shown in figure 1, the mixer/diffuser appliance according to the invention comprises a plurality of diffusers 1, each of which is designed to diffuse a perfume or fragrance in the room, which said diffusers are fitted to a common support 2, which may be a support plate, frame or the like, preferably designed to be secured to a back box 3. By way of example but not of limitation, the front surface 10 of the appliance could be made of glass or other material for appearance and protection purposes (Fig. 2).

Diffusers 1 are constituted by a system, which will now be described in greater detail, designed to suck in air from the room, cause it to flow over a preferably solid fragrance or perfuming product, and then convey it back into the room.

The perfuming product is preferably the solid type, for example in the form of pastilles (constituted by compressed powder mixed with a perfume or fragrance) or the like, contained in a cartridge shown as no. 4 in Fig. 1 which constitutes the subject of a separate application filed by the same applicant, each of which said cartridges is housed in a support frame 5 shown in Fig. 1, which is fitted to plate 2.

The front and back surfaces of each cartridge 4 are open or contain a series of openings designed in such a way that the air, conveyed by a fan 6, enters from the rear, flows over the perfumed pastilles, and then exits from the front.

The cartridges can be variously shaped according to the desired appearance and perfuming result.

Preferably, but not necessarily, the interior of the box into which the diffusers are inserted will be divided into sectors, one for each diffuser, by partition walls shown as no. 7 in figure 1.

Fans 6, driven by an electric motor which can be controlled at variable speed, are fitted to the back wall of frame 2.

A card, not shown in the figure, bears microprocessor devices of known type designed to control the system.

Each diffuser can also be associated with one or more LEDs or the like 8, which indicate its state of operation.

To regulate the emission of perfume as required, the internal microprocessor can be suitably programmed so that the diffuser/mixer device according to the invention is:
- fully independent, controlled with pushbuttons and switches not shown in the figure;
- controlled by a remote control not shown in the figure;
- connected to a dedicated external control unit with controls and timers;
- integrated with the regulation and control systems of the other electrical and electronic devices in the home (home automation control units).

The user chooses the cartridges with the preferred fragrances and inserts them into their frames, whereupon the appliance is ready to operate.

In these alternative ways the user can select the operating parameters, such as the fan speed and consequently the intensity of perfume emitted, or the period of time and corresponding intervals of operation.

It is consequently possible, for example, to activate one or more of the diffusers in succession for the desired period of time, for example to diffuse a deodorising fragrance, a relaxing perfume or a mixture of one or more of them at different times of day.

The appliance could be designed, by means of known technologies, so that the activation of one or more diffusers can be programmed at regular intervals, and so that the rotation speed of the various fans can be controlled to mix the different fragrances in different quantities, in order to obtain the perfume best suited to the user's tastes and requirements.

In conclusion, the device according to the invention provides great flexibility, as it enables the fragrance emitted to be modulated simply and effectively as required.

The different types of controls enable the operation of one or more diffusers to be activated manually, with LED 13 indicating their operation.

Different forms of embodiment could be devised within the ambit of the same solution idea.

For example, the device could include a different number of diffusers, or the diffusers could use a perfuming product other than compressed powder pastilles, while still remaining within the ambit of this invention.

## Claims

1. Appliance designed for mixing perfumed essences and diffusing them in a room, **characterised in that** it includes a common support or frame with which are associated a number of diffusers each comprising a fragrance container and means designed to diffuse said fragrance in the room simultaneously with the others, at an adjustable intensity.

2. Mixer/diffuser appliance as claimed in claim 1, **characterised in that** it includes a support for a plurality of diffusers, each of which comprises:
• a cartridge containing a fragrance to be diffused in the room;
• a fan designed to convey air so that it flows over the perfuming product contained in the cartridge;
• control means designed to allow independent, controlled operation of said diffusers.

3. Mixer/diffuser appliance as claimed in claim 2, **characterised in that** each of said diffusers is constituted by a support frame with a seating for the insertion of a cartridge containing the perfumed product and associated with a fan designed to direct an airstream against the perfumed product and then back into the room.

4. Mixer/diffuser appliance as claimed in claim 3, **characterised in that** it includes electronic means designed to control each diffuser independently of the others.

5. Mixer/diffuser appliance as claimed in each of the preceding claims, **characterised in that** it includes means designed to allow its insertion into a back box built into the wall.

6. Mixer/diffuser appliance as claimed in each of the preceding claims, **characterised in that** it includes means for connection to a control unit for programming the activation of the device.

7. Mixer/diffuser appliance as claimed in each of the preceding claims, **characterised in that** it includes a control for activation of the fan of each diffuser.

8. Mixer/diffuser appliance as claimed in each of the preceding claims, **characterised in that** it includes luminous means designed to display the operation of the fan of each diffuser.

9. Appliance for mixing and diffusing perfumed essences in a room, as described and illustrated.
